# EUROPEAN PATENT APPLICATION

(11) **EP 1 709 966 A1**
(43) Date of publication of application: **11.10.2006**
(21) Application number: 04807124.5
(22) Date of filing: 09.12.2004
(51) Int. Cl.: A61K 31/522, A61P 25/28, C07D 473/12, C07D 473/06

(54) **PREVENTIVE AND/OR THERAPEUTIC AGENT FOR HIGHER BRAIN DYSFUNCTION**

(30) Priority: 09.12.2003 JP 2003410432
(71) Applicant: KYOWA HAKKO KOGYO CO., LTD., Chiyoda-ku, Tokyo 100-8185 (JP)
(72) Inventor: KASE, Hiroshi, Koganei-shi Tokyo 184-0013 (JP); NAKAGAWA, Yutaka, Kanagawa 250-0034 (JP); SHIOZAKI, Shizuo KYOWA HAKKO KOGYO CO.,LTD, Shizuoka 411-8731 (JP); KOBAYASHI, Minoru KYOWA HAKKO KOGYO CO.,LTD, Shizuoka 411-8731 (JP); TOKI, Shinichiro KYOWA HAKKO KOGYO CO.,LTD, Shizuoka 411-8731 (JP); SENO, Naoki KYOWA HAKKO KOGYO CO.,LTD, Shizuoka 411-8731 (JP); IKEDA, Ken, Shizuoka 411-0942 (JP)
(74) Representative: Vossius & Partner
(86) International application number: PCT/JP2004/018765
(87) International publication number: WO 2005/056016

(57) **Abstract**

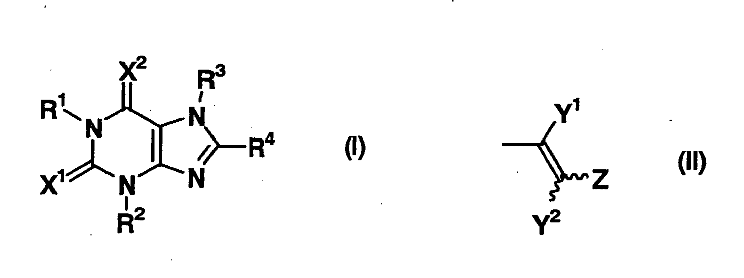

(wherein R¹, R² and R³ are the same or different, and represent a hydrogen atom, lower alkyl, lower alkenyl or lower alkynyl; R⁴ represents cycloalkyl, -(CH₂)ₙ-R⁵ or a group represented by the above formula (II) ; and X¹ and X² are the same or different, and represent an oxygen atom or a sulfur atom)

The present invention provides, for example, agents for preventing and/or treating higher brain dysfunction comprising, as an active ingredient, a xanthine derivative represented by the above formula (I) or a pharmaceutically acceptable salt thereof.

## Description

### Technical Field

The present invention relates to agents for preventing and/or treating higher brain dysfunctions comprising, as an active ingredient, a xanthine derivative or a pharmaceutically acceptable salt thereof.

### Background Art

The higher brain dysfunctions generally means that higher brain functions for performing the daily life, such as memory, thinking, recognition, action, learning, language and attention, are impaired by brain injuries due to various causes. Examples of those include the brain dysfunctions caused by brain injuries due to diseases, accidents or aging, such as (1) hemispatial neglect (visual perceptual defects,characterized by a failure to be aware of half of the space while the patients are aware of watching the whole space) ; (2) aphasia (Wernicke's aphasia (fluent aphasia), Broca' s aphasia (nonfluent aphasia) ; defect or loss of language function including "talking, hearing, reading or writing" relative to the comprehension or expression of words) ; (3) apraxia (ideokinetic apraxia, ideomotor apraxia; impairments characterized by difficulties in performing the intended movement and action, or the indicted movement and action even though their hands and legs can be moved); (4) agnosia (somatagnosia (anosognosia); disturbances in recognition of the body, for example, in which the patients do not recognize their own body parts belonging to themselves, consider the body parts being lost, or do not positively recognize occurrence of paralysis; visual agnosia; prosopagnosia; auditory agnosia; a lost of ability to recognize what is the object despite the capacity to identify its visual, auditory or tactile elements); (5) memory impairments (amnesia; impairments characterized by difficulties in memory and learning (particularly difficult in memory of new matter)); (6) executive dysfunction (impairments characterized by difficulties in doing a series of performances, that is, arrangement of information, planning, consideration of a process and practicing); (7) aprosexia (decrease of attentiveness and ability to concentrate) ; and (8) behavior and emotional impairments (referring to states such as excitation at trifling matters and acting impulsively; getting into a panic in a striking anxiety; and conversely decreasing spontaneity) [see; Merck Manual, 17th edition, Section 169, Total Rehabilitation, vol.11, p.605-608 (1983); Shin Seirikagaku Taikei (A Survey of New Physiological Science) 12, p.1-5 (1988), edited by Toshio Suzuki, Igaku-Shoin Ltd.].

The diseases causing the brain injuries which are causes of higher brain dysfunctions include, for example, head traumas (e.g., extradural hematoma, subdural hematoma, cerebral contusion, intracerebral hemorrhage, etc.), cerebrovascular accidents (e.g., intracerebral hemorrhage, cerebral infarction, cerebral apoplexy, hypoxic encephalopathy, subarachnoid hemorrhage, moyamoya disease, etc.), infections (e.g., encephalitis, AIDS encephalopathy, etc.), autoimmune diseases (e.g. systemic lupus erythematosus, nerve Behçet's disease, etc.), toxic diseases (e.g., alcoholism, carbon monoxide poisoning, drug abuse, etc.), brain tumor, and the like.

On the other hand, many of xanthine derivatives including the compound represented by the formula (I) which will be described later have been known to have, for example, anti-Parkinsonian action, central nerve exciting action, suppressive action on neurodegeneration, and the like (refer to Japanese Published Examined Patent Application No. 26, 516/1972; Japanese Published Unexamined Patent Application No. 211,856/1994; Japanese Published Unexamined Patent Application No. 239,862/1994; Japanese Published Unexamined Patent Application No. 016, 559/1994; WO 99/12546; etc.). They have been also known to have antagonistic action to adenosine A₂ receptor, antidepressive action, anti-asthma action, suppressive action for bone resorption, hypoglycemic action, suppressive action for thrombocytosis, and the like [WO92/06976, WO94/01114, WO95/23165, WO99/35147; Journal of Medicinal Chemistry (J. Med. Chem.), vol.34, p.1431 (1991); Journal of Medicinal Chemistry (J. Med. Chem.), vol.36, p.1333 (1993)].

### Disclosure of Invention

An object of the present invention is to provide agents for preventing and/or treating higher brain dysfunctions comprising, as an active ingredient, for example, a xanthine derivative or a pharmaceutically acceptable salt thereof, and the like.

The present invention relates to the following (1) to (14).
(1) An agent for preventing and/or treating higher brain dysfunction comprising, as an active ingredient, a xanthine derivative represented by formula (I): 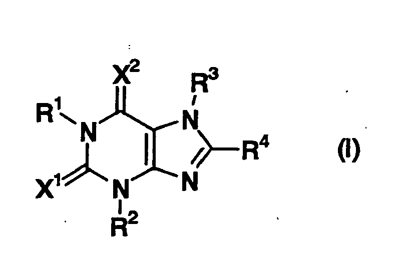 [wherein
   R¹, R² and R³ are the same or different, and represent a hydrogen atom, lower alkyl, lower alkenyl or lower alkynyl;
   R⁴ represents cycloalkyl, - (CH₂)ₙ-R⁵ (wherein R⁵ represents substituted or unsubstituted aryl, or substituted or a unsubstituted heterocyclic group; and n represents an integer of 0 to 4) or a group represented by formula (II): 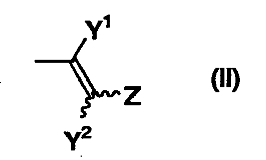 (wherein Y¹ and Y² are the same or different, and represent a hydrogen atom, halogen or lower alkyl; and Z represents substituted or unsubstituted aryl or a substituted or unsubstituted heterocyclic group);
   X¹ and X² are the same or different, and represent an oxygen atom or a sulfur atom]
   or a pharmaceutically acceptable salt thereof.
(2) The agent for preventing and/or treating higher brain dysfunction according to the above (1), wherein X¹ and X² each is an oxygen atom.
(3) The agent for preventing and/or treating higher brain dysfunction according to the above (1) or (2), wherein R⁴ is a group represented by formula (II) : 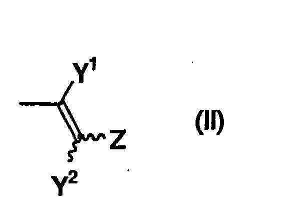 (wherein Y¹, Y² and Z each have the same meanings as defined above).
(4) The agent for preventing and/or treating higher brain dysfunction according to the above (3), wherein Y¹ and Y² are both hydrogen atoms.
(5) The agent for preventing and/or treating higher brain dysfunction according to the above (3) or (4), wherein Z is substituted or unsubstituted aryl or a group represented by formula (III): 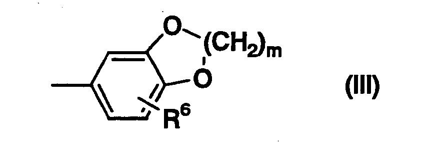 (wherein R⁶ represents a hydrogen atom, hydroxy, lower alkyl, lower alkoxy, halogen, nitro or amino; and m represents an integer of 1 to 3).
(6) The agent for preventing and/or treating higher brain dysfunction according to any one of the above (1) to (5), wherein the higher brain dysfunction is a higher brain dysfunction caused by brain injury.
(7) The agent for preventing and/or treating higher brain dysfunction according to the above (6), wherein the brain injury is a brain injury due to aging.
(8) The agent for preventing and/or treating higher brain dysfunction according to the above (6), wherein the brain injury is a brain injury due to a disorder selected from the group consisting of head trauma and cerebrovascular accident.
(9) The agent for preventing and/or treating higher brain dysfunction according to any one of the above (1) to (8), wherein the higher brain dysfunction is an impairment of higher brain function selected from the group consisting of memory, thinking, recognition, action and learning.
(10) The agent for preventing and/or treating higher brain dysfunction according to any one of the above (1) to (8), wherein the higher brain dysfunction is a brain dysfunction selected from the group consisting of agnosia, amnesia and apraxia.
(11) The agent for preventing and/or treating higher brain dysfunction according to any one of the above (1) to (8), wherein the higher brain dysfunction is a memory impairment.
(12) The agent for preventing and/or treating higher brain dysfunction according to any one of the above (1) to (8), wherein the higher brain dysfunction is a learning impairment.
(13) A method for preventing and/or treating higher brain dysfunction which comprises administering an effective amount of a xanthine derivative represented by formula (I): 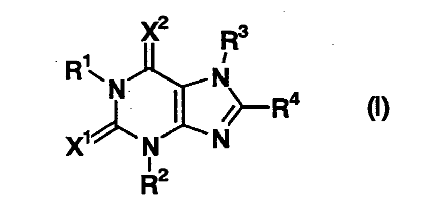 [wherein R¹, R², R³, R⁴, X¹ and X² each have the same meanings as defined above]
   or a pharmaceutically acceptable salt thereof.
(14) Use of a xanthine derivative represented by formula (I) : 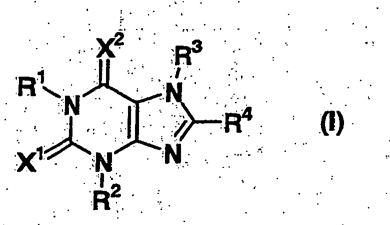 [wherein R¹, R², R³, R⁴, X¹ and X² each have the same meanings as defined above]
   or a pharmaceutically acceptable salt thereof for the manufacture of an agent for preventing and/or treating higher brain dysfunction.

In the present invention, "higher brain dysfunctions" means that "higher brain functions for performing the daily life, such as memory, thinking, recognition, action, learning, language and attention, are impaired by brain injuries due to various causes". Specifically, examples, of those include the "higher brain dysfunction" caused by "brain injuries" due to diseases, accidents or aging. More specifically, examples of those include the brain dysfunctions caused by brain injuries due to diseases, accidents or aging, such as (1) hemispatial neglect; (2) aphasia (Wernicke's aphasia (fluent aphasia), Broca's aphasia (nonfluent aphasia) and the like); (3) apraxia; (4) agnosia (somatagnosia, (anosognosia), visual agnosia, prosopagnosia, auditory agnosia and the like); (5) memory impairments (amnesia and the like); (6) executive dysfunction; (7) aprosexia; and (8) behavibr and emotional impairments. The diseases causing the "brain injuries" which are causes of these higher brain dysfunctions include, for example, head traumas (e.g., extradural hematoma, subdural hematoma, cerebral contusion, intracerebral hemorrhage, etc.), cerebrovascular accidents (e.g., intracerebral hemorrhage, cerebral infarction, cerebral apoplexy, hypoxic encephalopathy, subarachnoid hemorrhage, moyamoya disease, etc.), infections (e.g., encephalitis, AIDS encephalopathy, etc.), autoimmune diseases (e.g. systemic lupus erythematosus, nerve Behçet's disease, etc.), toxic diseases (e.g., alcoholism, carbon monoxide poisoning, drug abuse, etc.), brain tumor, and the like.

In particular, the "impairments of higher brain functions" which can be prevented and/or treated appropriately by agents for preventing and/or treating higher brain dysfunctions of the present invention include, for example, impairments of memory, thinking, action, learning and the like, and functions combined with one or more of them, that is, recognition, cognition, execution and the like. Among them, one or more of the impairments of higher brain functions selected from impairments of memory, thinking, action, learning, recognition, cognition and execution can be preferably prevented and/or treated.

Furthermore, in particular, the "higher brain dysfunctions", which can be prevented and/or treated suitably by agents for preventing and/or treating higher brain dysfunctions of the present invention, include "higher brain dysfunctions" caused by " brain injuries" due to diseases, accidents or aging as described above. Among them, hemispatial neglect, apraxia, agnosia, memory impairments, learning impairments, executive dysfunctions and the like can be suitably prevented and/or treated.

In the definition of each group in formula (I):
Examples of the lower alkyl and the lower alkyl moiety of the lower alkoxy include straight-chain or branched alkyl groups having 1 to 6 carbons, such as methyl, ethyl, propyl, isopropyl, butyl, isobutyl, sec-butyl, tert-butyl, pentyl, neopentyl and hexyl.
Examples of the lower alkenyl include straight-chain or branched alkenyl groups having 2 to 6 carbons, such as vinyl, allyl, methacryl, crotyl, 3-butenyl, 2-pentenyl, 4-pentenyl, 2-hexenyl and 5-hexenyl.
Examples of the lower alkynyl include straight-chain or branched alkynyl groups having 2 to 6 carbons, such as ethynyl, propargyl, 2-butynyl, 3-butynyl, 2-pentynyl, 4-pentynyl, 2-hexynyl, 5-hexynyl and 4-methyl-2-pentynyl.
Examples of the cycloalkyl include cycloalkyl groups having 3 to 8 carbons, such as cyclopropyl, cyclobutyl, cyclopentyl, cyclohexyl, cycloheptyl and cyclooctyl.

The halogen means an atom of fluorine, chlorine, bromine and iodine.

Examples of the aryl include those having 6 to 14 carbons, such as phenyl, naphthyl and anthryl.

Examples of the heterocyclic group include 5- or 6-membered monocyclic heterocyclic groups containing at least one atom selected from a nitrogen atom, an oxygen atom and a sulfur atom; bicyclic or tricyclic condenced-ring heterocyclic groups containing at least one atom selected from a nitrogen atom, an oxygen atom and a sulfur atom in which 3- to 8-membered rings are condensed and the like. Specific examples thereof include specifically, furyl, thienyl, pyrrolyl, pyranyl, thiopyranyl, pyridyl, pyrimidinyl, triazinyl, purinyl, pyrazinyl, pyridazinyl, benzimidazolyl, 2-oxobenozimidazolyl, benzotriazolyl, benzofuryl, benzothienyl, benzoxazolyl, benzothiazolyl, 1,3-benzodioxolyl, 1,4-benzodioxanyl, 3,4-dihydro-2H-1,5-benzodioxepinyl, indazolyl, indolyl, isoindolyl, quinolyl, isoquinolyl, phthalazinyl, naphthyridinyl, quinoxalinyl, pyrazolyl, quinazolinyl, cinnolinyl, triazolyl, tetrazolyl, imidazolyl, oxazolyl, isoxazolyl, thiazolyl, isothizaolyl, dihydroisoquinolyl, tetrahydroquinolyl and dihydrobenzopyranyl.

The substituted aryl and the substituted heterocyclic group have 1 to 3 substituents which are the same or different, such as lower alkyl, lower alkenyl, lower alkynyl, hydroxy, substituted or unsubstituted lower alkoxy, halogen, nitro, amino, lower alkylamino, di-lower alkylamino, trifluoromethyl, trifluoromethoxy, aralkyl, aralkyloxy, aryl, aryloxy, lower alkanoyl, lower alkanoyloxy, aroyl, aroyloxy, arylalkanoyloxy, carboxy, lower alkoxycarbonyl, lower alkylcarbamoyl, di-lower alkylcarbamoyl, sulfo, lower alkoxysulfonyl, lower alkylsulfamoyl and di-lower alkylsulfamoyl.

The lower alkyl moiety of the above-described lower alkyl, lower alkoxy, lower alkylamino, di-lower alkylamino, lower alkanoyl, lower alkanoyloxy, lower alkoxycarbonyl, lower alkylcarbamoyl, di-lower alkylcarbamoyl, lower alkoxysulfonyl, lower alkylsulfamoyl and di-lower alkylsulfamoyl has the same meaning as the above-described lower alkyl. The halogen, the lower alkenyl and the lower alkynyl have the same meanings as described above, respectively. Two lower alkyl moieties of the di-lower alkylamino, the di-lower alkylcarbamoyl and the di-lower alkylsulfamoyl may be the same or different. An aryl moiety of the aryl and the aryloxy is the same as the above-described aryl, and examples of the aralkyl moiety of the aralkyl and the aralkyloxy include benzyl, phenethyl and the like. Examples of an aroyl moiety in the aroyl and the aroyloxy include benzoyl, naphthoyl and the like. Examples of an arylalkyl moiety of the arylalkanoyloxy are benzyl, phenethyl and the like. Examples of the substituent (s) in the substituted lower alkoxy include hydroxy, lower alkoxy, halogen, amino, azido, carboxy, lower alkoxycarbonyl and the like. Herein, a lower alkyl moiety of the lower alkoxy and the lower alkoxycarbonyl has the same meaning as the.above-described lower alkyl, and the halogen has the same meaning as described above.

Hereinafter, a compound represented by formula (I) will be referred to as Compound (I).

Examples of the pharmaceutically acceptable salt of Compound (I) are pharmaceutically acceptable acid addition salt, metal salt, ammonium salt, organic amine addition salt, amino acid addition salt and the like.

Examples of the pharmaceutically acceptable acid addition salts of Compound (I) include an inorganic acid salt such as hydrochloride, sulfate and phosphate; and an organic acid salt such as acetate, maleate, fumarate, tartrate, citrate and methanesulfonate. Examples of the pharmaceutically acceptable metal salts include an alkali metal salt such as sodium salt and potassium salt; alkaline earth metal salt such as magnesium salt and calcium salt; aluminum salt; zinc salt and the like. Examples of the pharmaceutically acceptable ammonium salts include ammonium and tetramethylammonium. Examples of the pharmaceutically acceptable organic amine addition salt include an addition salt of morpholine or piperidine. Examples of the pharmaceutically acceptable amino acid addition salts include an addition salt of lysine, glycine or phenylalanine.

Compound (I) is able to be produced by a process disclosed in Japanese Published Examined Patent Application No. 26,516/1972; Journal of Medicinal Chemistry (J. Med. Chem.), vol. 34, p. 1431 (1991); Journal of Medicinal Chemistry(J. Med. Chem.), vol. 36, p. 1333 (1993) ; WO 92/06976; Japanese Published Unexamined Patent Application No. 211,856/1994; Japanese Published Unexamined Patent Application No. 239,862/1994; WO 95/23165; Japanese Published Unexamined Patent Application No. 16559/1994; WO 94/01114; WO 99/12546; WO 99/35147 and the like, or by a process similar thereto. The desired compound in each production process can be isolated and purified by a purifying method which has been commonly used in synthetic organic chemistry such as filtration, extraction, washing, drying, concentration, recrystallization and various chromatographies.

When it is desired to obtain a salt of Compound (I), in the case where Compound (I) is produced in the form of the salt, it can be purified as it is, but where it is produced in its free form, it can be converted into a salt, after being dissolved or suspended in an appropriate solvent followed by adding an appropriate acid or base.

Furthermore, Compound (I) and pharmaceutically acceptable salts thereof may exist in the form of adducts with water or various solvents, and these adducts are also used as the agents for preventing and/or treating higher brain dysfunctions of the present invention.

For some of Compounds (I), there may exist optical isomers and the like, and all possible isomers including them and mixtures thereof may be used as the agents for preventing and/or treating higher brain dysfunctions of the present invention.

Specific examples of Compound (I) are shown in Table 1.

**Table 1**

| **Compound No.** | |
|---|---|
| 1 | 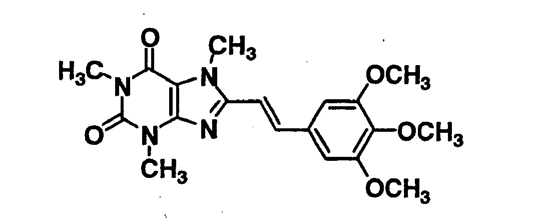 |
| 2 | 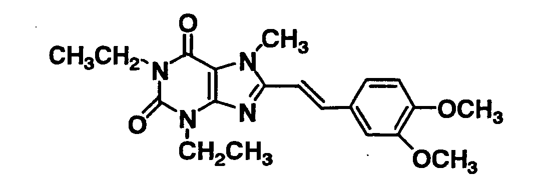 |
| 3 | 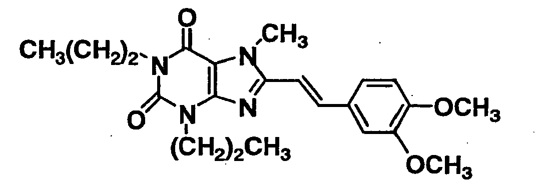 |
| 4 | 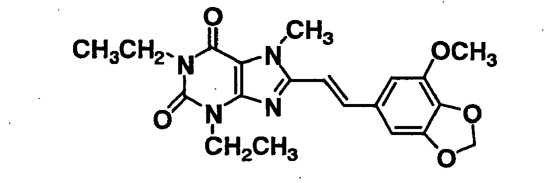 |

The effect of the present invention will be explained by the following Test Examples.

### Test Example 1: Test for learning of active avoidance

Experiment was carried with using 10 SD male rats (body weight: 220-280 g) for one group.

Using a shuttle box apparatus consisting of two boxes (TK-401S; Unicom), the rats were made to learn and train (escape training) conditioned avoidance behavior. In one trial in the escape training, an alarm stimulus by light and buzzer sound as a conditioned stimulus was given to the rats for 8 seconds. When the rat did not move into the next box within 4 seconds from the beginning of the alarm stimulus (avoidance behavior by alarm stimulus), an unconditioned stimulus was given to the rat by flowing an electric current of 3 mA on a floor grid for 4 seconds (learning-strengthening factor). Repetition of the trial was conducted at interval times of 5 seconds, and the escape training was repeated 50 trials/1 training/1 day for 10 days.

The test compound was used as a suspension in injectable distilled water containing 0.3% Tween-80 (by Otsuka Pharmaceutical Co., Ltd.; 0.3% Tween-80 solution) and orally administered to each of the rats at a dose of 10 mL/kg 1 hour before the test (Test compound-administered group). Separately, 0.3% Tween-80 solution alone was orally administered to each of the rats at a dose of 10 mL/kg 1 hour before the test (Solvent-administered group).

For analysis of the test results, various parameters were inputted in a personal computer (NEC; PC-9821Xe) and the avoidance rate (%)(average in 10 rats; the rats which took avoidance behavior by an alarm stimulus was regarded as successful escape; the avoidance rate when the rat succeeded in escaping all 50 trials was regarded as 100%) and the change of the total reaction latency (the time required for the completion of the whole trial) were compared with those of Solvent-administered group. The analysis was carried out by a Steel-test. Table 2 shows the results.

**Table 2**

| Administered group (Dose mg/kg) | Avoidance rate (%) | Time required for completion of the whole trial (sec) |
|---|---|---|
| Solvent | 90.8 | 99.1 |
| Compound 2 (0.03) | 96.2 | 81.2 |
| Compound 2 (0.3) | 97.2 | 64.9 * |
| Compound 2 (3.0) | 99.2 ** | 65.3 ** |

| | | |
|---|---|---|
| Steel-test: *= p < 0.05; ** = p < 0.01 | | |

From the above results, the followings became clear.

In Compound 2-administered group, the avoidance rate was higher than that of Solvent-administered group (90.8%). In addition, in Compound 2 (3.0 mg/kg)-administered group, the avoidance rate significantly increased (p<0.01; Steel-test).

On the other hand, in Compound 2-administered group, the whole trial was completed within a shorter period of time than that of Solvent-administered group (99.1 seconds). In Compound 1(0.3 mg/kg)-administered group and Compound 1(3.0 mg/kg)-administered group, the time required for the completion of the whole trials was significantly reduced (p<0.05 and p<0.01, respectively; Steel-test).

### Test Example 2: Test for learning of passive avoidance

Experiment was carried out using 32 albino female rats (body weight: about 100 g) for one group.

Test for learning of passive avoidance was conducted in a test box (40 cm × 31 cm × 29 cm) in which a stainless steel grid was spread on the floor so that an electric shock of 1.1 mA can be give randomly. In the test box, a plastic plate (15 cm × 5 cm) of 0.5 cm thickness was placed at a corner to cover the floor; and the test box comprised a mechanism that when a rat placed on the plate steps down on the floor grid, the rat results in receiving an electric shock. Usually, the rat attacked by an electric shock immediately gets back on the plastic plate but tries to get down on the floor; then, the rat is attacked by an electric shock again. Usually, the rat gradually changes its behavior with repetition of the shock (learning and training), resulting in staying on the plastic plate (passive avoidance behavior).

After the learning and training, the rats keeping the memory were selected and used for the test based on the memory-keeping test as mentioned below. Each of the rat was placed on the plastic plate, and from this time as the starting point, (1) in the case in which the rat got down on the floor within 60 seconds and (2) in the case in which the rat did not resist to get down on the floor by softly pushing after lapse of 60 seconds, the rat was judged to keep no memory of learning for passive avoidance behavior.

The test compound was used as a suspension thereof in distilled water containing 0.3% Tween-80; and scopolamine was used as a solution thereof in distilled water (by Sigma).

To each of the rats which learned the passive avoidance behavior, immediately after learning, 1 mg/kg (2 mL/kg volume) of scopolamine (subcutaneous administration) and 2 mL/kg volume of a test compound (oral administration) was administered simultaneously (Test compound-administered group); and 2 hours after administration, the rats were evaluated whether they kept memory of learning of the passive avoidance behavior. Separately, to each of the rats which learned the passive avoidance behavior, immediately after learning, 2 mL/kg volume of 0.3% Tween-80 solution alone was orally administered, and the rats were referred to as Solvent-administered group; while in the same manner, the rats to which 1 mg/kg of scopolamine (2 mL/kg volume) each were administered subcutaneously, were referred to as Scopolamine-administered group.

The test results were analyzed by means of a Two-tailed Chi Square test by comparing the rate of the rats exhibiting the passive avoidance behavior with that of Scopolamine-administered group. Table 3 shows the results.

**Table 3**

| Administered group (Dose mg/kg) | Rate of the rats exhibiting passive avoidance behavior (%) |
|---|---|
| Solvent | 26/32* (81.30) |
| Scopolamine | 4/32 (12.5%) |
| Scopolamine + Compound 2 (0.3) | 17/32* (53.1%) |
| Scopolamine + Compound 2 (3.0) | 24/32* (75.0%) |
| Scopolamine + Compound 2 (30) | 27/32* (84.4%) |

| | |
|---|---|
| Two-tailed Chi Square test: * = p < 0.05 | |

From the above results, the followings became clear.

In Scopolamine-administered group in which impairment of memory process was caused by administration of scopolamine, the rate of the rats exhibiting the passive avoidance behavior was remarkably decreased in comparison with that of Solvent-administered group. On the other hand, in the group to which Compound 2 was simultaneously administered with scopolamine (Test compound-administered group), the rate of the rats exhibiting the passive avoidance behavior was significantly increased in comparison with that of Scopolamine-administered group (p < 0.05: Two-tailed Chi Square test).

### Test Example 3: Maze-learning test in rats prepared by bilateral ligation of the common carotid arteries

The test was carried out according to the methods described in the following references.
(1) Acta. Neuropathol. 87, p.484-492 (1994)
(2) Brain Res., vol.729, p.55-65 (1996)
(3) Neuroscience, vol.79, p.1039-1050 (1997)
(4) Nichi-yakuri-shi, vol.113, p.85-95 (1999)
(5) Stroke, vol.26, p.1415-1422 (1995)
(6) No-shinkei, vol.49, p.639-644 (1997)
(7) Jpn. J. Pharmacol., vol.75, p.443-446 (1997)

Experiment was carried out using 10 Wistar female rats (body weight 210-310 g) for one group.

Under anesthesia with pentobarbital, the necks of rats were incised and the bilateral common carotid arteries were ligated (ligated rats). Separately, the necks of rats were incised but the bilateral common carotid arteries were not ligated (sham operation rats). Under anesthesia with pentobarbital, the rat was fixed on a brain-fixing apparatus for rat (Narishige; SR-6). Hydrogen electrodes (Unique Medical; UHE-100, IS needle type) were inserted into a brain coordinate (A2.0, L2.0, D-2.0). Hydrogen indifferent electrode (Unique Medical) was placed on the rat's neck. An artificial respiration was applied to the rat through a respiratory apparatus (Shinano Seisakusyo; SN-480-7), through which about 40 mL of hydrogen gas was forcibly inhaled 2 or 3 times at intervals of about 15 minutes.

In the maze-learning test, a grayish radial maze of vinyl chloride was used. The maze was set 50 cm above the floor and consisted of 8 radially extended lanes (12 cm × 60 cm) and a central platform, where a hollow (3 cm diameter, 1 cm in depth) were made at each end of the lane for placing milk as a reward.

Running for the maze-learning test was performed twice. In the first running, milk for reward was placed in all hollows of 8 lanes, among which 4 lanes were blocked by blocks so that the rat could not enter into the lanes. In the first running, the rat was able to enter the maze freely. When the rat returned to the platform after obtaining 4 hollows of milk was constrained by a cylinder placed on the platform, during which the blocks on the 4 lanes were removed. Ten seconds after constraint of the rat, the cylinder was removed and the second running was performed. In the second running, the rat was able to obtain milk placed in the hollows of remaining 4 lanes. At that time, the frequency of selection of the lanes required for obtaining the 4 hollows of milk was recorded up to 16th round of selection.

The maze-learning test was started from 4 weeks after the bilateral ligation of the common carotid arteries. The first running and the second running were conducted as one trial; and the training of 2 trials a day was made at intervals of about 15 minutes and for 10 consecutive days. The average of the selection frequency in the first and second trials was regarded as a learning result. The lanes on which blocks were placed in the first running were determined according to each rat and were maintained constantly during the test period.

The test compound was used as a suspension thereof in injectable distilled water containing 0.5% methylcellulose (MC) (Otsuka Pharmaceutical Co.) and was administered orally to each of the above ligated rats at a dose of 10 mL/kg volume one hour before the start of the first trial (Test compound-administered group). Separately, to each of the above sham operation rats and each of the above ligated rats, 0.5% MC solution alone was orally administered at a dose of 10 mL/kg volume one hour before the start of the first trial, respectively, and were referred to Sham operation group and Solvent-administered group, respectively.

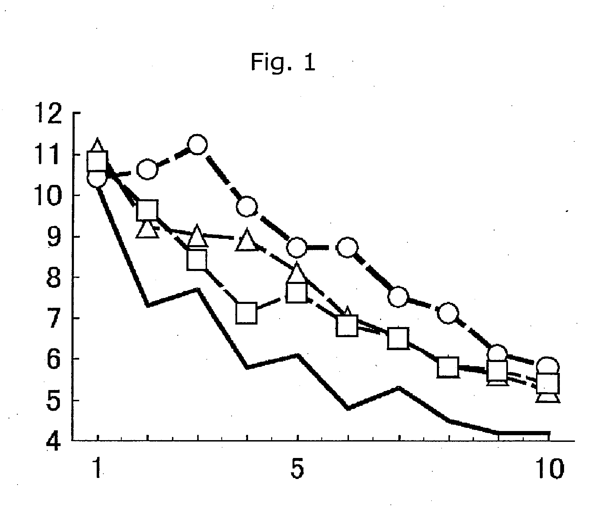

Test results were analyzed by means of Bonferroni-type multiple testing procedures by comparing the frequency of the lane selection (Selection frequency of the lane) required for obtaining 4 hollows of milk in the second running of the maze-learning test in Solvent-administered group. Fig.1 shows the results.

From the above-mentioned results, the followings became clear.

In Solvent-administered group, the lane selection frequency was significantly increased in comparison with Sham operation group, indicating decrease of the learning result (p <0.01; Bonferroni-type multiple testing procedures). In Test compound-administered group to which Compound 1 (1 mg/kg) and Compound 1 (3 mg/kg) were administered respectively, decrease of the learning results caused by bilateral ligation of the common carotid arteries was significantly improved, respectively (p < 0. 025 and p < 0.005; Bonferroni-type multiple testing procedures).

### Test Example 4: Test for learning a delayed alternating problem (non-matching to sample)

The test was carried out in a manner similar to the method as described in Drug Dev. Res., 35, p.83-95 (1996).

Experiment was carried out using 10 Wistar (Han) male rats (body weight 200-250 g) for one group.

A Skinner box (30 cm × 25 cm × 30 cm) equipped with 3 pull-type lever (at the center, left and right sides) connected to a feeding vessel (supplying 45 mg diet pellet) was used in the experiment. The control of the test condition and the data accumulation were automatically conducted by connecting with a MED.PC program system.

Each rat received learning and training of lever pushing, and thereafter learning and training of a delayed alternating problem. In the learning and training of lever pushing, the rat learned and was trained so that the diet could be obtained by pushing any one of the center, left and right pull-type levers. In the subsequent learning and training of delayed alternating problem, the rat learned and was trained that the diet could be obtained by pushing any one of the left and right levers at first, and then the pushed lever was withdrawn; then, 5 seconds after that, both of the left and right levers were presented, but the diet was supplied only when the opposite side lever of the one pushed previously was pushed.

The time of presentation of each lever was fixed at 20 seconds, and if the rat did not push the lever within that time, the lever was withdrawn, and the subsequent trial started 10 seconds after that.

The test compound was used as a suspension thereof in injectable distilled water containing 0.5% methylcellulose (MC) (Otsuka Pharmaceutical Co.) and was administered orally to each of the rats which learned and trained the lever pushing at a dose of 10 mL/kg volume once a day from the start of learning and training of a delayed alternating problem continuously for a week (Test compound-administered group). Separately, to each of the rats which learned and trained the lever pushing, 0.5% MC solution alone was orally administered at a dose of 10 mL/kg volume once a day from the start of learning and training of a delayed alternating problem continuously for 1 week, and the rats were referred to as Solvent-administered group.

The accuracy rate for the reaction was indicated by the rate (%) of correctly selecting the left or right lever (average of 10 rats), and the test results were analyzed by one-way ANOVA-Student's t test. Table 4 shows the results.

**Table 4**

| Administered group (dose mg/kg) | Accuracy Rate for the reaction everyday (%) (Mean ± Standard deviation) | | | Accuracy rate for the reaction in a week(%) (Mean ± Standard deviation) |
|---|---|---|---|---|
| | 1st day | 3rd day | 5th day | |
| Solvent | 48.3±2.9 | 39.4±2.9 | 47.7±2.1 | 45.4±1.6 |
| Compound 2 (0.3) | 49.7±2.1 NS | 49.4±3.4* | 60.0±3.0** | 50.6±1.5* |

| | | | | |
|---|---|---|---|---|
| Student's t test: NS = Not Significant; * = p < 0.05; ** = p < 0.01 | | | | |

From the above-mentioned results, the followings became clear.

In the delayed alternating problem test, the accuracy rate for the reaction of Compound 2-administered group significantly increased in comparison with that of Solvent-administered group. The accuracy rate for the reaction after a week (Accuracy rate for the reaction in a week) significantly increased to 50.6 ± 1.5(%) in contrast to that of Solvent-administered 45.4 ± 1.6(%).

From the results of Test Examples 1 to 4 as mentioned above, it was elucidated that Compounds (I) or a pharmaceutically acceptable salts thereof have effects of improving and/or enhancing higher brain functions such as memory, learning, thinking, action, cognition, recognition and execution. In other words, it was shown that Compounds (I) or a pharmaceutically acceptable salts thereof are useful as agents for preventing and/or treating of higher brain dysfunctions (for example, hemispatial neglect, apraxia, agnosia, memory impairments, learning impairments, executive dysfunctions and the like, caused by brain injuries due to diseases, accidents or aging).

Compound (I) or a pharmaceutically acceptable salt thereof may be used either as it is or in various pharmaceutical dosage forms. The pharmaceutical composition of the present invention may be manufactured by a uniform mixing of Compound (I) or a pharmaceutically acceptable salt thereof as an active ingredient in an effective dose with a pharmaceutically acceptable carrier. It is preferred that such a pharmaceutical composition is in a unit dosage form suitable for the administration such as rectal administration or oral or parenteral (including subcutaneous, intravenous and intramuscular) administration.

In preparing a composition in an orally administering form, any useful pharmaceutically acceptable carrier may be used. In the case of oral liquid preparation such as suspension and syrup, it may be manufactured using water, saccharide such as sucrose, sorbitol and fructose, glycol such as polyethylene glycol and propylene glycol, oil such as sesame oil, olive oil and soybean oil, antiseptic agent such as p-hydroxybenzoate, flavor such as strawberry flavor and peppermint, etc. In the case of diluted powder, pill, capsule and tablet, it may be prepared using excipient such as lactose, glucose, sucrose and mannitol, disintegrating agent such as starch and sodium alginate, lubricant such as magnesium stearate and talc, binder such as polyvinyl alcohol, hydroxypropyl cellulose and gelatin, surfactant such as fatty acid ester, plasticizer such as glyceline, and the like. Tablets and capsules are the most useful unit agents being administered per os because their administration is easy. In the manufacture of tablets and capsules, a solid pharmaceutical carrier is used.

Injections preparation can be prepared using a carrier comprising distilled water, salt solution, glucose solution or a mixture of brine and glucose solution, or the like. In that case, it is prepared as solution, suspension or dispersion using an appropriate adjuvant according to the conventional method.

Compound (I) or a pharmaceutically acceptable salt thereof can be administered orally in the above-described pharmaceutical dosage form or parenterally as injections. Although effective dose and administering frequency thereof vary depending upon administration form, age and body weight of a patient, symptom, etc., it is appropriate to administer 1 to 100 mg/60 kg/day or, preferably, 1 to 20 mg/60 kg/day once daily or several times a day.

### Brief Description of Drawing

Fig.1 shows the effect of Compound 1 in the maze-learning test in rats prepared by bilateral ligation of the common carotid arteries. The ordinate indicates the frequency of the lane selection (Selection frequency of the lanes) required for obtaining the 4 hollows of milk in the second running in the maze-learning test. The abscissa indicates the number of days elapsed (days) after the initiation of the test. Each plot on the graph has the following significances.
   - ― :: Sham operation group
   - -○- :: Solvent-administered group
   - -△- :: Compound 1 (1 mg/kg)-administered group
   - -□- :: Compound 1 (3 mg/kg)-administered group

### Best Mode for Carrying Out the Invention

Embodiments of the present invention are illustrated in detail below referring to examples.

### Example 1: Tablets

Tablets comprising the following composition are prepared by a conventional method.

The compound 1 (40 g), 286. 8 g of lactose and 60 g of potato starch are mixed and 120 g of a 10% aqueous solution of hydroxypropyl cellulose is added thereto. The mixture is kneaded by a conventional method, granulated, dried and subj ected to particle size selection to give granules for making into tablets. Magnesium stearate (1.2 g) is added thereto and mixed therewith and subjected to tabletting using a tabletting machine (RT-15 manufactured by Kikushisha) having punches with 8 mm diameter to give tablets (each tablet contained 20 mg of the active ingredient).

| Prescription | |
|---|---|
| Compound 1 | 20 mg |
| Lactose | 143.3 mg |
| Potato starch | 30 mg |
| Hydroxypropyl cellulose | 6 mg |
| Magnesium stearate | 0.6 mg |
| | 200 mg |

### Example 2: Capsule preparations

Capsule preparations comprising the following composition are prepared by a conventional method.

The compound 2 (200 g), 995 g of Avicel and 5 g of magnesium stearate are mixed by a conventional method. The mixture is filled in hard capsules No. 4 (capacity of one capsule is 120 mg) using a capsule filling machine (type LZ-64; manufactured by Zanasi) to prepare capsule preparations (each capsule contained 20 mg of the active ingredient).

| Prescription | |
|---|---|
| Compound 2 | 20 mg |
| Avicel | 99.5 mg |
| Magnesium stearate | 0.5 mg |
| | 120 mg |

### Example 3: Injection preparations

Injection preparations comprising the following composition are prepared by a conventional method.

The compound 3 (1 g) is dissolved in 100 g of pure soybean oil and 12 g of pure yolk lecithin and 25 g of glycerol for injection are added thereto. The mixture is made 1, 000 mL using distilled water for injection by a conventional method followed by kneading and emulsifying. The resulting dispersion is subjected to an aseptic filtration using a membrane filter of a disposable type of 0.2 µm and each 2 mL thereof is aseptically filled in a glass vial to prepare injection preparations (each vial contained 2 mg of the active ingredient).

| Prescription | |
|---|---|
| Compound 3 | 2 mg |
| Pure soybean oil | 200 mg |
| Pure yolk lecithin | 24 mg |
| Glycerol for injection | 50 mg |
| Distilled water for injection | 1.72 mL |
| | 2.00 mL |

### Example 4: Suppositories for anus

A preparation for rectal administration comprising the following composition is prepared by a conventional method.

Witepsol ™ H15 (manufactured by Dynamite Nobel) (678.8 g) and 290. 9 g of Witepsol ™ E75 (manufactured by Dynamite Nobel) are melted at 40 to 50°C. The compound 4 (2.5 g), 13. 6 g of potassium primary phosphate and 14.2 g of sodium secondary phosphate are uniformly mixed with the above and dispersed therein. After that, the mixed/dispersed product is filled in suppository molds made of plastics followed by gradual cooling to prepare suppositories for anus (each preparation contained 2.5 mg of the active ingredient).

| Prescription | |
|---|---|
| Compound 4 | 2.5 mg |
| Witepsol ™ H15 | 678.8 mg |
| Witepsol ™ E75 | 290.9 mg |
| Potassium primary phosphate | 13.6 mg |
| Sodium secondary phosphate | 14.2 mg |
| | 1,000 mg |

### Industrial Applicability

The present invention provides agents for preventing and/or treating higher brain dysfunctions comprising, as an active ingredient, for example, a xanthine derivative or a pharmaceutically acceptable salt thereof.

## Claims

1. An agent for preventing and/or treating higher brain dysfunction comprising, as an active ingredient, a xanthine derivative represented by formula (I): 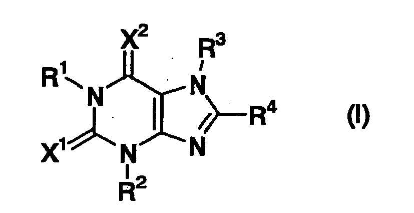 [wherein
R¹, R² and R³ are the same or different, and represent a hydrogen atom, lower alkyl, lower alkenyl or lower alkynyl;
R⁴ represents cycloalkyl, -(CH₂)ₙ-R⁵ (wherein R⁵ represents substituted or unsubstituted aryl, or substituted or a unsubstituted heterocyclic group; and n represents an integer of 0 to 4) or a group represented by formula (II): 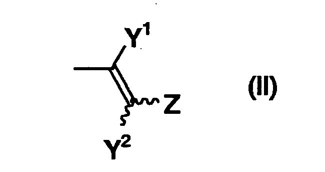 (wherein Y¹ and Y² are the same or different, and represent a hydrogen atom, halogen or lower alkyl; and Z represents substituted or unsubstituted aryl or a substituted or unsubstituted heterocyclic group);
X¹ and X² are the same or different, and represent an oxygen atom or a sulfur atom]
or a pharmaceutically acceptable salt thereof.

2. The agent for preventing and/or treating higher brain dysfunction according to claim 1, wherein X¹ and X² each is an oxygen atom.

3. The agent for preventing and/or treating higher brain dysfunction according to the claim 1 or 2, wherein R⁴ is a group represented by formula (II): 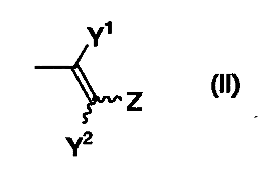 (wherein Y¹, Y² and Z each have the same meanings as defined above).

4. The agent for preventing and/or treating higher brain dysfunction according to claim 3, wherein Y¹ and Y² are both hydrogen atoms.

5. The agent for preventing and/or treating higher brain dysfunction according to claim 3 or 4, wherein Z is substituted or unsubstituted aryl or a group represented by formula (III) : 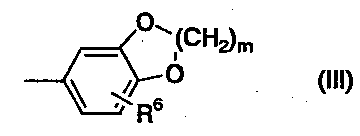 (wherein R⁶ represents a hydrogen atom, hydroxy, lower alkyl, lower alkoxy, halogen, nitro or amino; and m represents an integer of 1 to 3).

6. The agent for preventing and/or treating higher brain dysfunction according to any one of claims 1 to 5, wherein the higher brain dysfunction is a higher brain dysfunction caused by brain injury.

7. The agent for preventing and/or treating higher brain dysfunction according to claim 6, wherein the brain injury is a brain injury due to aging.

8. The agent for preventing and/or treating higher brain dysfunction according to claim 6, wherein the brain injury is a brain injury due to a disorder selected from the group consisting of head trauma and cerebrovascular accident.

9. The agent for preventing and/or treating higher brain dysfunction according to any one of claims 1 to 8, wherein the higher brain dysfunction is an impairment of higher brain function selected from the group consisting of memory, thinking, recognition, action and learning.

10. The agent for preventing and/or treating higher brain dysfunction according to any one of claims 1 to 8, wherein the higher brain dysfunction is a brain dysfunction selected from the group consisting of agnosia, amnesia and apraxia.

11. The agent for preventing and/or treating higher brain dysfunction according to any one of claims 1 to 8, wherein the higher brain dysfunction is a memory impairment.

12. The agent for preventing and/or treating higher brain dysfunction according to any one of claims 1 to 8, wherein the higher brain dysfunction is a learning impairment.

13. A method for preventing and/or treating higher brain dysfunction which comprises administering an effective amount of a xanthine derivative represented by formula (I): 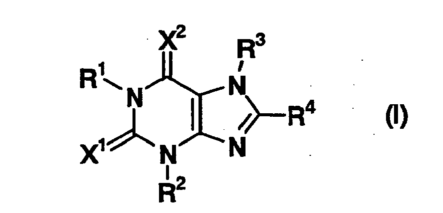 [wherein R¹, R², R³, R⁴, X¹ and X² each have the same meanings as defined above]
or a pharmaceutically acceptable salt thereof.

14. Use of a xanthine derivative represented by formula (I) : 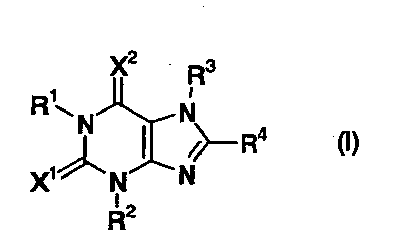 [wherein R¹, R², R³, R⁴, X¹ and X² each have the same meanings as defined above]
or a pharmaceutically acceptable salt thereof for the manufacture of an agent for preventing and/or treating higher brain dysfunction.
